# EUROPEAN PATENT APPLICATION

(11) **EP 4 079 295 A1**
(43) Date of publication of application: **26.10.2022**
(21) Application number: 20902732.5
(22) Date of filing: 16.12.2020
(51) Int. Cl.: A61K 9/16, A61K 9/00, A61K 31/502

(54) **COMPOSITION HAVING IMPROVED SOLUBILITY AND BIOAVAILABILITY OF OLAPARIB**

(30) Priority: 20.12.2019 KR 20190171683
(71) Applicant: Samyang Holdings Corporation, Seoul 03129 (KR)
(72) Inventor: LIM, Hye Jung, Seongnam-si Gyeonggi-do 13490 (KR); PARK, Sang Yeob, Yongin-si Gyeonggi-do 16875 (KR); LEE, Sa Won, Seongnam-si Gyeonggi-do 13554 (KR)
(74) Representative: Schrell, Andreas
(86) International application number: PCT/KR2020/018469
(87) International publication number: WO 2021/125797

(57) **Abstract**

The present invention relates to an oral composition comprising: Olaparib; and a polymethacrylate copolymer, and having improved solubility and bioavailability, and a method for preparing same.

## Description

### TECHNICAL FIELD

The present invention relates to an oral composition comprising Olaparib, which is a poorly water-soluble drug, with enhanced solubility and bioavailability, and a method for preparing the same.

### BACKGROUND ART

Olaparib is a phthalazinone derivative drug and the first PARP inhibitor approved in 2014, and it has been used for treatment of breast cancer and ovarian cancer. Olaparib is weak acidic with pKa 12.07 and is a poorly water-soluble substance with very low water solubility (0.1 to 0.13 mg/ml), and its transmittance is not so high and it is known as falling in BCS class 4. Such low solubility and bioavailability of the active drug ingredient make the drug formulation problematic, and so various methods for improvement such as crystal form change or salt form preparation, etc. have been tried.

Lynparza^{™} capsule which is a commercial formulation product, uses Gelucire^{™} 44/14 which is a lipid excipient, to improve the drug solubility. However, it has the drug content of 10 % in the formulation wherein 50 mg of Olaparib is filled in hard capsule of 0 size, and thus in case of 400 mg therapeutic dose, 8 capsules should be taken at once inconveniently.

Accordingly, in order to improve medication compliance of patients, it is necessary to reduce the single dosing unit by increasing bioavailability and simultaneously increasing the drug content in formulation, and thus various studies thereon are in progress.

### CONTENTS OF THE INVENTION

### PROBLEMS TO BE SOLVED

The first purpose of the present invention is to provide a solid comprising Olaparib with enhanced solubility (water solubility) and bioavailability.

The other purpose of the present invention is to provide an oral composition comprising Olaparib with enhanced solubility and bioavailability.

Another purpose of the present invention is to provide a method for preparing an oral solid formulation comprising Olaparib with enhanced solubility and bioavailability.

### TECHNICAL MEANS

One aspect of the present invention relates to a solid dispersion comprising Olaparib and polymethacrylate copolymer.

In an embodiment, the Olaparib is Olaparib monohydrate.

In an embodiment, the polymethacrylate copolymer is a cationic polymer with dimethyl-aminoethyl methacrylate.

In an embodiment, the polymethacrylate copolymer is poly(butyl methacrylate-co-(2-dimethylaminoethyl) methacrylate-co-methyl methacrylate).

In an embodiment, the polymethacrylate copolymer has a weight average molecular weight of 3,000 to 200,000 g/mole.

In an embodiment, the solid dispersion comprises the polymethacrylate copolymer in an amount of 0.05 to 10 parts by weight, based on 1 part by weight of Olaparib.

In an embodiment, the solid dispersion further comprises D-α-tocopheryl polyethylene glycol succinate.

In an embodiment, the solid dispersion further comprises D-α-tocopheryl polyethylene glycol succinate in an amount of 0.001 to 2 parts by weight, based on 1 part by weight of Olaparib.

In an embodiment, the solid dispersion is obtained by spray drying.

In an embodiment, the solid dispersion is amorphous.

The other aspect of the present invention relates to a method for preparing a solid dispersion comprising a step of dissolving or dispersing Olaparib together with polymethacrylate copolymer in a solvent, and then removing the solvent from the resulting solution of dispersion liquid.

In an embodiment, the removal of the solvent is conducted by spray drying.

Another aspect of the present invention relates to an oral composition comprising the solid dispersion and one or more pharmaceutically acceptable additives.

Still another aspect of the present invention relates to a method for preparing an oral composition comprising a step of mixing the solid dispersion and one or more pharmaceutically acceptable additives.

### EFFECT OF THE INVENTION

The Olaparib composition as disclosed in the present invention is an oral formulation capable of making the drug amorphized stably and increasing the solubility and dissolution rate, and thereby improving *in vivo* absorption ratio of the drug, and as compared with commercial product, it can reduce use of additive and thus increase drug content in the formulation, and thereby reduce the number of dosing unit taken at once so that the medication compliance of patients can be improved. In addition, advantages are expected in terms of the productivity such as preparation and packaging, etc. through application of composition and production process more favorable for formulation.

### BRIEF EXPLANATION OF THE DRAWINGS

Figure 1 represents the XRPD patterns of the solid dispersions prepared in Examples 1 to 3, respectively, and Olaparib API (active pharmaceutical ingredient), showing that Olaparib API itself used for preparing the solid dispersions was crystalline whereas the Olaparib contained in all solid dispersions prepared in Examples 1 to 3 was amorphous.
Figure 2 represents the elution patterns of the tablets prepared in Examples 5 to 8 and Comparative Example 1 in pH 1.2 solution.
Figure 3 represents the elution patterns of the tablets prepared in Example 5 and Comparative Examples 1 and 4 in water (distilled water, DW) and in pH 6.8 solution.

### PREFERRED MODE FOR CARRYING OUT THE INVENTION

### Definition of terms

Unless explicitly described otherwise, some terms used in the present entire specification can be defined as follows.

In the present entire specification, unless mentioned especially otherwise, "comprising" or "containing" refers to comprising a certain constitutional element (or constitutional component) without special limitation, and it is not interpreted as excluding addition of other constitutional element (or constitutional component).

### Discovery of technical means

In order to improve solubility of poorly water-soluble drug, it is possible to apply various methods including modification of the drug itself such as preparation of salt, crystal form, prodrug, etc., preparation as micro or nano emulsion, solid dispersion, etc., drug particle size reduction to micro or nano size, micelle formation, etc. In such improvement of property, since the characteristics of drugs such as solubility, pH, pKa, melting point, molecular weight, functional group, 3-dimensional structure, crystallinity, transmittance, absorption site, degradability, effective drug dose, etc. are different drug by drug, a single technology cannot be commonly applied to all drugs and thus it is necessary to find a manner working for a specific drug.

The present inventors have made much efforts to evaluate influence of various materials on the solubility of Olaparib, and finally discovered a solid dispersion having excellent effect in improving solubility and bioavailability of Olaparib and an oral composition using the same.

### Detailed explanation

One aspect of the present invention provides a solid dispersion comprising Olaparib and polymethacrylate copolymer.

"Olaparib" may be Olaparib base (free base drug with no separate salt), or its pharmaceutically acceptable salt or its isomer, or a mixture thereof. Also, in each case it may form various hydrates, and in each case it may form various crystal forms. For example, it may be a crystal form of Olaparib hydrate or anhydrous Olaparib, or an amorphous form thereof, or a mixture thereof.

In an embodiment, Olaparib may be Olaparib monohydrate.

Olaparib may be in various forms, and for example, it may be in a micronized form in the size of several to several tens of micrometers, more preferably a micronized form in the size of several micrometers, and still more preferably a nanoscaled forms to the size of hundreds of nanometers. As the particle size of the drug becomes smaller, the dissolution rate becomes higher, and thus the solubility and bioavailability can be increased.

In an embodiment, the polymethacrylate copolymer may be a cationic polymer with dimethyl-aminoethyl methacrylate, and more concretely, it may be poly(butyl methacrylate-co-(2-dimethylaminoethyl) methacrylate-co-methyl methacrylate). The poly(butyl methacrylate-co-(2-dimethylaminoethyl) methacrylate-co-methyl methacrylate) may be a polymer comprising each of the monomers with 1:2:1 molar ratio.

As a concrete example, the polymethacrylate copolymer may be Eudragit^{®} E PO (Evonik, Germany) or Eudragit ^{®} E 100 (Evonik, Germany). Eudragit^{®} E polymer is a conventional coating base material, and used when it is needed to protect the drug from moisture, or if the patient's medication compliance is poor, for the purpose of alleviating the patient's reluctance to take by encapsulating the sensitive drug or masking the flavor and smell of the drug, and providing smooth and shiny surface so that the patient can swallow the drug easily. Whereas, in the present invention, it is used as a polymer for improving solubility and bioavailability of Olaparib.

The polymethacrylate copolymer may have a weight average molecular weight of 3,000 to 200,000, or 5,000 to 150,000, or 10,000 to 100,000, or 20,000 to 80,000, or 37,000 to 57,000 g/mole. If the weight average molecular weight of the polymethacrylate copolymer is less than 3,000 g/mole, the effect of improving water solubility may be low, and if the weight average molecular weight is greater than 200,000 g/mole, disintegration may be delayed.

There is no special limitation to the state of the polymethacrylate copolymer, but it may be is a state of granule or powder.

In an embodiment, the amount of the polymethacrylate copolymer comprised in the solid dispersion may be, based on 1 part by weight of Olaparib, 0.05 part by weight or more, 0.1 part by weight or more, 0.5 part by weight or more, 1 part by weight or more, or 1.5 parts by weight or more, and it also may be 10 parts by weight or less, 9 parts by weight or less, 8 parts by weight or less, 7 parts by weight or less, 6 parts by weight or less, 5 parts by weight or less, 4 parts by weight or less, or 3 parts by weight or less, but it is not limited thereto.

For example, the solid dispersion may comprise the polymethacrylate copolymer comprised in an amount of 0.05 to 10 parts by weight, 0.1 to 6 parts by weight, 0.1 to 5 parts by weight, 0.5 to 4 parts by weight, 1 to 3 parts by weight, or 1.5 to 3 parts by weight, based on 1 part by weight of Olaparib. If the amount of the polymethacrylate copolymer in the solid dispersion is less than the above lower limit, the effect of improving solubility and bioavailability of Olaparib may be insufficient, and if it is greater than the above upper limit, the formulation (e.g., capsule or tablet) becomes too big, which may cause discomfort to patients when taking it.

In an embodiment, the solid dispersion may further comprise hydrophilic polymer.

The hydrophilic polymer may be, for example, one or more selected from the group consisting of polyvinylcaprolactam-polyvinyl acetate-polyethylene glycol graft copolymer (e.g., Soluplus ^{®}), hydroxypropylmethylcellulose (HPMC), polyvinylpyrrolidone (PVP), polyvinyl acetate (PVA), carboxymethyl cellulose (sodium salt and calcium salt), ethylcellulose, methylcellulose, hydroxyethylcellulose, ethylhydroxyethylcellulose, hydroxypropylcellulose (HPC), L-HPC (low-substituted HPC), polyvinyl alcohol, polymer of acrylic acid or salt thereof, vinylpyrrolidone-vinylacetate copolymer (e.g., Kollidon ^{®} VA64, BASF), Polycoat IR, gelatin, guar gum, partially hydrolyzed starch, alginate, xanthan, and mixtures thereof, but it is not limited thereto. Or, the hydrophilic polymer may be polyvinylcaprolactam-polyvinyl acetate-polyethylene glycol graft copolymer (e.g., Soluplus ^{®}), hydroxypropylmethylcellulose (HPMC) or a mixture thereof.

In an embodiment, the hydrophilic polymer may be comprised in the solid dispersion, for example, in an amount of 0.1 to 10 parts by weight, 0.1 to 5 parts by weight, 0.1 to 3 parts by weight, to 0.5 to 3 parts by weight, or 1 to 3 parts by weight, based on 1 part by weight of Olaparib. If the amount of the hydrophilic polymer is less than the above lower limit, the degree of improving solubility and bioavailability of Olaparib may be insufficient, and if it is greater than the above upper limit, the formulation (e.g., capsule or tablet) becomes too big, which may cause discomfort to patients when taking it.

In an embodiment, the solid dispersion may further comprise D-α-tocopheryl polyethylene glycol succinate (Vitamin E TPGS or TPGS). In this case, excellent effect can be obtained for improving solubility and dissolution rate of Olaparib.

The TPGS may be comprised in the solid dispersion, for example, in an amount of 0.001 to 2 parts by weight, 0.001 to 1 part by weight, 0.005 to 1 part by weight, or 0.01 to 0.1 part by weight, based on 1 part by weight of Olaparib. If the amount of the TPGS is less than the above lower limit, the degree of improving solubility and bioavailability of Olaparib may be insufficient and thus the effect may be lowered, and if it is greater than the above upper limit, the coating layer becomes too sticky, which may cause difficulty in film formation and storage as well as preparation.

In an embodiment, the solid dispersion may be a spray-dried one. The solid dispersion is not a form of coated inactivated core, and thus it may be used in preparing tablet so that more Olaparib can be contained in a tablet having a size as small as possible, and it may prevent a phenomenon wherein the disintegration rate and elution ratio rapidly increase or decrease.

In an embodiment, the solid dispersion is amorphous. If the solid dispersion is amorphous, since the solubility increases as compared with crystal form, there is advantage of further increased *in vivo* absorption ratio.

The term "solid dispersion" used in the present invention refers to a form wherein the material to be dispersed (generally, drug) in crystal or amorphous form is dispersed in an amorphous polymer (continuous phase). The solid dispersion generally comprises drug and polymer, and in some cases, it may further comprise other additive(s) for drug such as surfactant, plasticizer, disintegrating agent, etc.

In an embodiment, the solid dispersion may not comprise surfactant.

In an embodiment, the solid dispersion may further comprise surfactant.

The surfactant may be, for example, anionic surfactant. The anionic surfactant may be sodium dodecyl sulfate, sodium lauryl sulfate, sodium N-lauroyl sarcosylate, salt of N-long chain acyl glutamic acid, sucrose fatty acid ester, polyoxyethylene hydrogenated castor oil, sorbitan fatty acid ester, copolymer of polyoxyethylene and polyoxypropylene, or a combination thereof, or it may be sodium dodecyl sulfate.

The other aspect of the present invention provides a method for preparing a solid dispersion comprising a step of dissolving or dispersing Olaparib together with polymethacrylate copolymer in a solvent, and then removing the solvent from the resulting solution of dispersion liquid.

In an embodiment, the solvent used to prepare the solid dispersion may be water-miscible organic solvent, water or a mixture thereof, and it may include supercritical fluid. The water-miscible organic solvent may be, for example, selected from the group consisting of alcohol, acetone, tetrahydrofuran, acetic acid, acetonitrile, dioxane, and combinations thereof, but it is not limited thereto. The water-miscible organic solvent may be, for example, a lower alcohol having 1 to 5 carbon atoms, acetone, or a mixture thereof, or it may be ethanol, acetone or a mixture thereof, or it may be ethanol.

The removal of the solvent may be conducted by using vacuum drying, spray drying, tray drying, freeze drying or other drying technique, and in an embodiment, the removal of the solvent is conducted by spray drying. In this case, for the spray drying, spray dryer, fluid bed dryer, or other dryer may be used.

The solid dispersion prepared as above may be mixed with other additional components and may be powdered or granulized, or tableted by tableting the mixture, or encapsulated by filling into a capsule.

Another aspect of the present invention provides an oral composition with enhanced solubility and bioavailability, comprising the solid dispersion and one or more pharmaceutically acceptable additives.

Still another aspect of the present invention provides a method for preparing an oral composition with enhanced solubility and bioavailability, comprising a step of mixing the solid dispersion and one or more pharmaceutically acceptable additives.

The oral composition may be used for the treatment of cancer.

In an embodiment, the cancer is selected from breast cancer and ovarian cancer.

The oral composition may be, for example, an oral solid formulation in tablet, capsule, granule, or powder form, and particularly it may be tablet or capsule form. In an embodiment, the oral composition is tablet.

The oral composition may comprise the solid dispersion in an amount of 0.01 to 0.9 part by weight, 0.01 to 0.8 part by weight, 0.05 to 0.8 part by weight, 0.1 to 0.7 part by weight, 0.3 to 0.7 part by weight, or 0.4 to 0.7 part by weight, based on 1 part by weight of the total composition. If the oral composition comprises the solid dispersion in an amount greater than the above range, it is difficult to formulate due to low tableting properties, and if the amount is less than the above range, the formulation becomes too big, which may cause discomfort to patients when taking it.

The pharmaceutically acceptable additive may be, for example, diluent, disintegrating agent, lubricant, surfactant, pH adjusting agent, coating agent, or combination thereof. In an embodiment, the oral composition may comprise diluent, disintegrating agent, lubricant, pH adjusting agent, or combination thereof.

The diluent may be, for example, one or more selected from the group consisting of lactose (anhydrous or hydrated, e.g., monohydrate), cellulose powder, microcrystalline cellulose, silicified microcrystalline cellulose, starch, dicalcium phosphate, tricalcium phosphate, magnesium trisilicate, mannitol, maltitol, sorbitol, xylitol, lactose, dextrose, maltose, sucrose, glucose, fructose, maltodextrin, and mixtures thereof, but it is not limited thereto. Preferably, lactose, mannitol, microcrystalline cellulose or a mixture thereof may be selected. Most preferably, microcrystalline cellulose or mannitol may be selected.

In this case, the diluent may be used in an amount of, for example, 1 to 70 parts by weight, 5 to 50 parts by weight, or 10 to 40 parts by weight, based on 100 parts by weight of the total tablet weight. If the amount of the diluent is less than the above lower limit, it is difficult to formulate due to low tableting properties, and if the amount is greater than the above upper limit, the tablet becomes too big, which may cause discomfort to patients when taking it.

The disintegrating agent may be, for example, one or more selected from the group consisting of croscarmellose sodium (CrosCMC-Na), carboxymethylcellulose, crospovidone (crosslinked polyvinylpyrrolidone), L-HPC (low-substituted hydroxypropylcellulose), starch (wheat, rice, corn or potato starch), sodium carboxymethyl starch, sodium glycolate of potato starch, partially hydrolyzed starch, and mixtures thereof, but it is not limited thereto. Preferably, it may be croscarmellose sodium (CrosCMC-Na) or starch sodium glycolate, L-HPC (low-substituted hydroxypropyl cellulose) or a mixture thereof.

In this case, the disintegrating agent may be used in an amount of, for example, 1 to 30 parts by weight, 2 to 20 parts by weight, 2 to 10 parts by weight, or 2 to 6 parts by weight, based on 100 parts by weight of the total tablet weight. If the amount of the disintegrating agent is less than the above lower limit, there may be a problem of dissolution rate delay due to disintegration rate delay, and if the amount is greater than the above upper limit, there may be a problem with productivity such as tableting disorder.

The lubricant may be, for example, one or more selected from the group consisting of magnesium stearate, fumaric acid, stearic acid, calcium stearate, sodium stearyl fumarate, polyethylene glycol, starch (wheat, rice, corn or potato starch), talc, highly dispersed (colloidal) silica, magnesium oxide, magnesium carbonate, glyceryl behenate, glyceryl monostearate, silicon dioxide, calcium silicate, magnesium silicate and mixtures thereof, but it is not limited thereto. Preferably, it may be magnesium stearate. As the lubricant, magnesium stearate may be used alone.

In this case, the lubricant may be used in an amount of, for example, 0.1 to 3 parts by weight, 0.2 to 3 parts by weight, 0.5 to 2.5 parts by weight, or 0.5 to 2 parts by weight, based on 100 parts by weight of the total tablet weight. If the amount of the lubricant is less than the above lower limit, there may be a problem with productivity such as tableting disorder, and if the amount is greater than the above upper limit, there may be dissolution rate delay or a problem with productivity.

The pH adjusting agent collectively refers to a compound that changes the balance of acidity-basicity in the formulation, and concretely it may be carboxylic acid, acrylic acid, tartaric acid, citric acid, malic acid, maleic acid, fumaric acid, acetic acid, succinic acid, lactic acid, succinic acid, malonic acid, glutaric acid or a mixture thereof. The pH adjusting agent may be used to improve solubility difference of the polymethacrylate copolymer according to the water solvent pH.

In an embodiment, the pH adjusting agent is an acidic compound including organic acid having carboxylic acid group, and preferably it may be dibasic acid having two carboxylic functional group, but it is not limited thereto. The dibasic acid may be, for example, maleic acid, tartaric acid, citric acid, fumaric acid or a mixture thereof, and in an embodiment, the pH adjusting agent is fumaric acid, maleic acid or a mixture thereof.

In an embodiment, the pH adjusting agent may be used in a proper amount according to the properties of each component such as pKa and stability, and for example, it may be used in an amount of 0.01 to 5 parts by weight, 0.05 to 3 parts by weight, 0.1 to 1 part by weight, 0.1 to 0.5 part by weight, 0.1 to 0.3 part by weight, 0.1 to 0.25 part by weight, or 0.3 to 0.8 part by weight, based on 1 part by weight of the polymethacrylate copolymer. If the amount of the pH adjusting agent is less than the above lower limit, the effect of reducing solubility difference according to pH change may be insufficient, and if the amount is greater than the above upper limit, there may be an influence on stability of the formulation in light of the characteristics of strongly reactive material.

In other embodiment, the oral composition may not comprise surfactant.

The coating agent may be, for example, as hydrophilic polymer, one or more selected from the group consisting of polyvinylpyrrolidone (PVP), polyvinyl acetate (PVA), hydroxypropylmethylcellulose (HPMC), carboxymethylcellulose (sodium salt and calcium salt), ethylcellulose, methylcellulose, hydroxyethylcellulose, ethylhydroxy ethyl cellulose, hydroxypropyl cellulose (HPC), L-HPC (low substituted HPC), polyvinyl alcohol, polymer of acrylic acid or salt thereof, vinyl pyrrolidone-vinyl acetate copolymer (e.g., Kollidon) (registered trademark) VA64, BASF), Polycoat IR, gelatin, guar gum, partially hydrolyzed starch, alginate, xanthan, and mixtures thereof, but it is not limited thereto. Preferably, it may be polyvinyl acetate (PVA) or hydroxypropylmethylcellulose (HPMC).

The coating agent may be used in an amount of, for example, 0.2 to 10 parts by weight, preferably 1 to 7 parts by weight, more preferably 3 to 5 parts by weight, based on 100 parts by weight of the tablet before coating (uncoated tablet). If the amount of the coating agent is less than the above lower limit, there may be a problem of incomplete coverage of entire surface of uncoated tablet with the coating agent, and if the amount is greater than the above upper limit, there may be a problem of excessive delay of elution rate.

The solid dispersion may be mixed with other additional components and may be powdered or granulized, or tableted by tableting the mixture, or encapsulated by filling into a capsule.

By using the solid dispersion, an oral composition may be prepared, for example, in case of tablet preparation, by utilizing a preparation method comprising a step of mixing the solid dispersion, diluent, and pH adjusting agent; a step of further mixing disintegrating agent; a step of further mixing lubricant; and a step of tableting the mixture, but it is not limited thereto.

Examples are provided below in order to facilitate understanding of the present invention. However, the following examples are only to illustrate the present invention, and the scope of the present invention is not limited thereby in any manner.

### [EXAMPLES]

### Examples 1 to 4: Preparation of solid dispersion comprising Olaparib

Olaparib hydrate (crystal form) (as monohydrate, same as below), Eudragit E PO or Soluplus, and TPGS (D-α-tocopheryl polyethylene glycol succinate) with the amounts shown in Table 1 below were dissolved completely in EtOH, and the solvent was removed by spray drying, to obtain a solid dispersion comprising Olaparib.

**[Table 1]**

| | Example 1 | Example 2 | Example 3 | Example 4 |
|---|---|---|---|---|
| Olaparib (g) | 2 | 2 | 2 | 2 |
| Eudragit E PO (g) | 5 | 4 | 2 | - |
| Soluplus (g) | - | - | - | 5 |
| TPGS (g) | 0.1 | 0.1 | 0.1 | 0.1 |
| EtOH (ml) | 200 | 200 | 200 | 200 |

### Examples 5 to 8: Preparation of tablet comprising Olaparib solid dispersion

Tablets of Examples 5 to 8 were prepared by using the solid dispersions prepared in Examples 1 to 4, respectively. Concretely, the solid dispersion and microcrystalline cellulose and fumaric acid were mixed first according to the compositional ratio shown in Table 2 below, then croscarmellose sodium was added thereto and mixed, then magnesium stearate as lubricant was added thereto and mixed, and then the mixture was tableted to prepare a tablet with total weight of 600 mg comprising 100 mg of Olaparib. The elution pattern of the tablet under in vivo pH condition was confirmed.

### Comparative Example 1

Lynparza^{™} (Olaparib 50 mg) capsule currently on market was used.

**[Table 2]**

| | Example 5 | Example 6 | Example 7 | Example 8 | Comparative Example 1 |
|---|---|---|---|---|---|
| Olaparib solid dispersion (100 mg as Olaparib) | 363.7 | 311.6 | 207.5 | 363.7 | Lynparza capsule (50 mg as Olaparib) |
| Microcrystalline cellulose (mg) | 177.3 | 229.4 | 333.4 | 177.3 | |
| Fumaric acid (mg) | 29.0 | 29.0 | 29.0 | 29.0 | |
| Croscarmellose sodium (mg) | 24.0 | 24.0 | 24.0 | 24.0 | |
| Magnesium stearate (mg) | 6.0 | 6.0 | 6.0 | 6.0 | |
| Total (mg) | 600.0 | 600.0 | 600.0 | 600.0 | |

### Comparative Examples 2 and 3: Preparation of Olaparib simple mixture (weight ratio of 1:2.5)

As a comparative example to Example 1, 2.0 g of Olaparib hydrate (crystal form), 5.0 g of Eudragit E PO and 0.1 g of TPGS were simply mixed to prepare Comparative Example 2.

Also, as a comparative example to Example 4, 2.0 g of Olaparib hydrate (crystal form), 5.0 g of Soluplus and 0.1 g of TPGS were simply mixed to prepare Comparative Example 3.

The prepared Comparative Examples 2 and 3 were used in solubility analysis of Test Example 1.

### Comparative Example 4: Preparation of Olaparib solid dispersion tablet not comprising organic acid

As a comparative example to Example 5, the additives except for fumaric acid (pH adjusting agent) in the solid dispersion-containing tablet composition of Example 5 were mixed according to the compositional ratio shown in the following table, and the mixture was tableted to obtain 100 mg Olaparib tablet.

**[Table 3]**

| | Comparative Example 4 |
|---|---|
| Olaparib solid dispersion (100 mg as Olaparib) | 363.7 |
| Microcrystalline cellulose (mg) | 206.3 |
| Croscarmellose sodium (mg) | 24.0 |
| Magnesium stearate (mg) | 6.0 |
| Total (mg) | 600.0 |

### Test Example 1: Comparison of drug solubility of Olaparib and polymer between simple mixture and solid dispersion state

To 20 ml of distilled water and pH 1.2 buffer, each of the solid dispersions of Example 1 and 4 and the simple mixtures of Comparative Examples 2 and 3 corresponding to 400 mg of Olaparib was added, and stirred in an incubator at 37°C with 100 rpm for 1 hour. The test solution was filtered with RC syringe filter (0.2 µm) and 1 mL of the filtered solution was taken and diluted with 2 mL of the following diluent solution, and then the amount was measured according to Olaparib amount analysis method. The results are shown below.

### Amount analysis condition

1) Diluent solution preparation
   Diluent solution - water (DW): acetonitrile (CAN)=1:1
2) Operation
   Detector: UV absorption spectrometer 254 nm
   Column: Nova-Pak C18 Column, 60Å, 4 µm, 3.9 mm × 150 mm
   Flow rate: 1.2 ml/min
   Injection amount: 5 µl
   Column temperature: 35°C
   Run time: 16 min
   Mobile phase

**[Table 4]**

| Minute | DW (mobile phase A) | ACN(mobile phase B) |
|---|---|---|
| 0 | 95 | 5 |
| 7 | 65 | 35 |
| 10 | 5 | 95 |
| 11 | 95 | 5 |
| 16 | 95 | 5 |

**[Table 5]**

| | Solubility (mg/ml) | |
|---|---|---|
| | pH 1.2 | water |
| Olaparib | 0.10 | 0.10 |
| Olaparib, Eudragit E simple mixture (Comparative Example 2) | 0.25 | 0.10 |
| Olaparib, soluplus simple mixture (Comparative Example 3) | 0.61 | 0.62 |
| Olaparib, Eudragit E solid dispersion (Example 1) | 2.46 | 0.17 |
| Olaparib, Soluplus solid dispersion (Example 4) | 0.88 | 1.00 |

### Test Example 2: Measurement of drug crystallinity by XRPD analysis

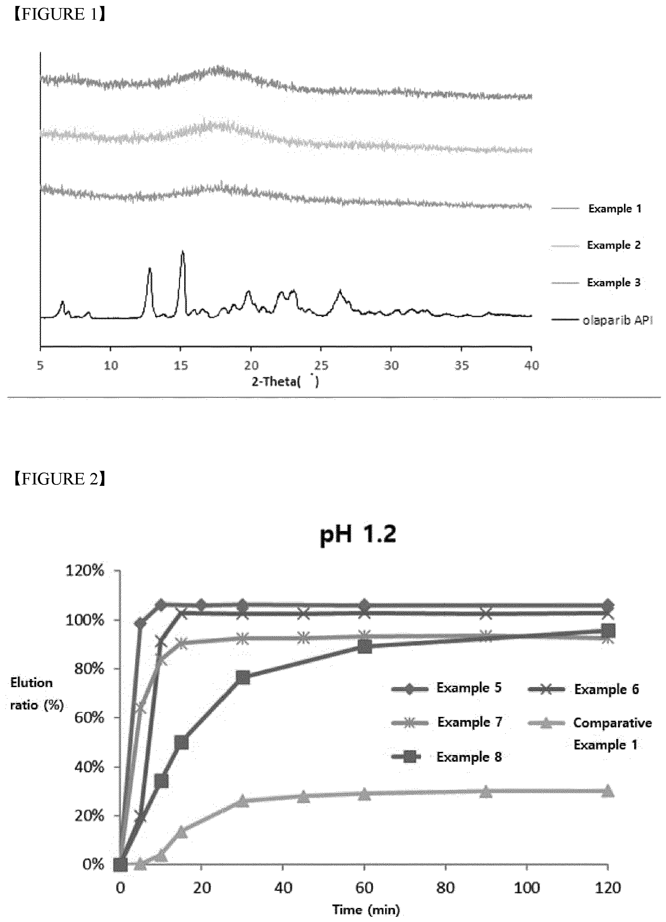

For Examples 1 to 3 and the raw material Olaparib, XRPD analysis was made under the analysis condition shown below, and the crystallinity of Olaparib in the solid dispersion was shown in Figure 1. As a result of the test, it was confirmed that the solid dispersions of Examples 1 to 3 did not exhibit the crystallinity peak of Olaparib observed in the raw material and thus Olaparib therein existed in amorphous form.

**[Table 6]**

| Instrument | High Power X-Ray Diffractometer (D/max-2500V/PC) |
|---|---|
| 2 theta range | 2 θ = 1.2 - 146 deg. |
| Sample amount | 5~10 mg |

| Analysis condition | |
|---|---|
| Scan range | 2 θ = 2 - 60° |
| Scan speed | 0.24°/sec |
| Step size | 0.02° |
| Power | 45 kv 150 mA |

### Test Example 3: Comparison of stability

In order to evaluate stability of the solid dispersion of Example, an accelerated test was conducted. The solid dispersion prepared in Example 1 was put into aluminum bag pouch together with a drying agent and sealed, and then stored under accelerated storage condition of 40°C, 75% RH for 3 months. XRPD analysis was made at the time of before storing the sample, 1 month after the storage and 3 months after the storage, to confirm the crystallinity of the drug and measure the elution ratio. The results are shown below.

### Elution condition

The amount of solid dispersion corresponding to 400 mg of Olaparib was weighed exactly and an elution test was conducted under the condition below. A sample was taken at the time of 60 minutes and the elution ratio was measured through HPLC analysis. In case of Comparative Example 1, low elution ratio was confirmed already at initial stage and crystal form was generated, and thus no further test was progressed.
Test solution: pH 1.2 buffer 900 ml
Stirring speed: 100 rpm
Temperature: 37°C

**[Table 7]**

| | Initial stage | | 1 month | | 3 months | |
|---|---|---|---|---|---|---|
| | Elution ratio (%) | XRPD¹⁾ | Elution ratio | XRPD | Elution ratio | XRPD |
| Example 1 | 101.1 | N/D ²⁾ | 103.9 | N/D | 101.8 | N/D |
| Comparative Example 1 | 29.1 | Peak observed | - | - | - | - |

| | | | | | | |
|---|---|---|---|---|---|---|
| 1) Crystallinity peak observed in XRPD 2) N/D: Not detected | | | | | | |

### Test Example 4: Elution test of Olaparib solid dispersion tablet

To the tablets of Examples 5 to 8 and Comparative Example 1 (Lynparza^{™} capsule formulation), elution ratios according to time were measured under pH 1.2 solution condition which was the same as gastric juice. The results are shown in Figure 2.

### Elution condition

Test solution: pH 1.2
Stirring speed: 100 rpm
Temperature: 37°C
Standard time of elution: 5 minutes, 10 minutes, 15 minutes, 30 minutes, 45 minutes, 60 minutes, 90 minutes, 120 minutes
Analysis method: HPLC method

### Test Example 5: Elution test of Olaparib solid dispersion tablet containing Eudragit E in neutral pH solution

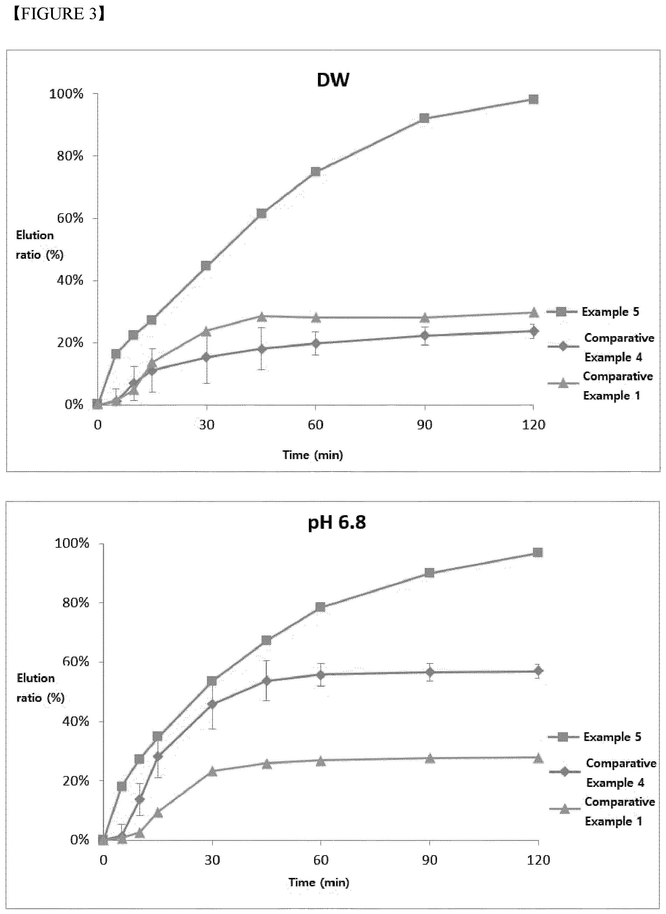

To the tablets of Example 5 and Comparative Example 4, elution ratios according to time were measured in water and under pH 6.8 condition. The results are shown in Figure 3.

### Elution condition

Test solutions: water, pH 6.8 solution
Stirring speed: 100 rpm
Temperature: 37°C
Standard time of elution: 5 minutes, 10 minutes, 15 minutes, 30 minutes, 45 minutes, 60 minutes, 90 minutes, 120 minutes
Analysis method: HPLC method

### Test Example 6: Pharmacokinetic (PK) test of Olaparib solid dispersion

In order to compare *in vivo* behavior of Example 5 and Comparative Example 1, pharmacokinetic test was conducted with SD rats as test animal. 15 SD rats were divided into groups with 5 rats per group, and the first group was classified as control group and the second group was classified as example group. For each group, the composition was dispersed to have 4 mg/ml concentration as Olaparib, and administered orally to the test animals maintained in a fasted state in 10 mg/kg dose. After the administration, the blood samples were taken with predetermined time interval up to 24 hours, and serums were separated from the blood samples and frozen for storage, and analyzed with LC/MS/MS device for concentration analysis to obtain the drug concentrations in blood according to time. From the data, AUC (area under serum concentration-time curve) and Cₘₐₓ (the maximum concentration in blood) were obtained, and the results are shown in Table 8 below.

**[Table 8]**

| | AUC (ng/ml•h) | Cₘₐₓ (ng/ml) |
|---|---|---|
| Comparative Example 1 | 236.2 | 94.3 |
| Example 5 | 469.3 | 587.6 |

From the results of pharmacokinetic test, it was confirmed that the solid dispersion composition comprising Olaparib according to the present invention exhibited higher AUC and Cₘₐₓ than the conventional hard capsule formulation currently on market comprising Olaparib, and thus it has excellent pharmacokinetic characteristics as compared with the formulation currently on market.

## Claims

1. A solid dispersion comprising Olaparib and polymethacrylate copolymer.

2. The solid dispersion of claim 1, wherein the polymethacrylate copolymer is poly(butyl methacrylate-co-(2-dimethylaminoethyl) methacrylate-co-methyl methacrylate).

3. The solid dispersion of claim 1, wherein the polymethacrylate copolymer is comprised in an amount of 0.05 to 10 parts by weight, based on 1 part by weight of Olaparib.

4. The solid dispersion of claim 1, further comprising D-α-tocopheryl polyethylene glycol succinate.

5. The solid dispersion of claim 4, wherein D-α-tocopheryl polyethylene glycol succinate is comprised in an amount of 0.001 to 2 parts by weight, based on 1 part by weight of Olaparib.

6. The solid dispersion of claim 1, which is amorphous.

7. A method for preparing a solid dispersion comprising a step of dissolving or dispersing Olaparib together with polymethacrylate copolymer in a solvent, and then removing the solvent from the resulting solution of dispersion liquid.

8. The method for preparing a solid dispersion of claim 7, wherein the removal of the solvent is conducted by spray drying.

9. An oral composition comprising a solid dispersion of any one of claims 1 to 6, and one or more pharmaceutically acceptable additives.

10. The oral composition of claim 9, further comprising diluent, disintegrating agent, lubricant, pH adjusting agent, or combination thereof.

11. A method for preparing an oral composition comprising a step of mixing a solid dispersion of any one of claims 1 to 6, and one or more pharmaceutically acceptable additives.
